# EUROPEAN PATENT APPLICATION

(11) **EP 1 939 160 A1**
(43) Date of publication of application: **02.07.2008**
(21) Application number: 06256457.0
(22) Date of filing: 19.12.2006
(51) Int. Cl.: C07C 6/10, C07C 9/08, C07C 9/10, C07C 9/14

(54) **Process for converting natural gas into higher alkanes**

(71) Applicant: BP OIL INTERNATIONAL LIMITED, Sunbury-on-Thames, Middlesex TW16 7BP (GB)
(72) Inventor: The designation of the inventor has not yet been filed
(74) Representative: De Kezel, Eric

(57) **Abstract**

The present invention relates to a process for converting natural gas into higher alkanes having at least 3 carbon atoms (C₃₊), preferably at least 4 carbon atoms (C₄+). The process comprises: a stage (1) comprising contacting natural gas with a metal catalyst (C1) capable of producing, in contact with alkane, reactions involving the splitting and recombining of carbon-carbon and/or carbon-hydrogen and/or carbon-metal bonds, so as to form a mixture (M1) comprising ethane and hydrogen, optionally a stage (2) comprising separating ethane from the mixture (M1) so as to isolate the ethane, a stage (3) for metathesis of the ethane, comprising contacting ethane isolated in stage (2) or the mixture (M1) obtained in stage (1) with a metal catalyst (C2) conforming to the same definition as that of the catalyst (C1), either identical to or different from the catalyst (C1), so as to form a mixture (M2) comprising higher alkanes (C₃₊), preferably (C₄₊), and methane, and a stage (4) comprising separating the higher alkanes (C₃₊), preferably (C₄₊), so as to isolate said higher alkanes. The catalysts (C1) and (C2) can be chosen from metal catalysts capable of alkane metathesis, particularly from supported metal clusters and preferably from metal hydrides, organo-metallic compounds and organometallic hydrides, in particular supported on, especially grafted onto, a solid support.

## Description

The present invention relates to a process for converting natural gas into higher alkanes having in particular at least 3 carbon atoms (C₃₊), preferably at least 4 carbon atoms (C₄₊).

Natural gas is very widely distributed in the world, and its deposit sites are often very far removed from one another and from the consumption areas. It follows that natural gas often has to be transported over long distances. It is however difficult to transport because of its gaseous nature. Natural gas can nevertheless be liquefied and transported more easily in liquid form. However, the energy and capital costs in achieving the liquefaction conditions and in then transporting the natural gas in liquid form are very high and may be often prohibitive for the exploitation of natural gas deposits, particularly ones of low capacity.

A solution would be to convert the natural gas into higher alkanes which would be liquid under standard temperature and pressure conditions or conditions relatively close to the latter, and which could therefore be easily transportable in liquid form. Such a conversion process would have to be simple and realisable at relatively low costs, enabling it to be installed directly on the natural gas deposit sites. This is the solution which the present invention proposes.

Processes for converting methane into liquid fuels by an oxidising route have already been known for a long time. The processes comprise first of all a reforming stage for converting the methane into a mixture of carbon monoxide and hydrogen, also called "synthesis gas" or "syngas", and then one or more stages for converting said mixture into gasoline and other easily transportable fuels. However, said processes require a great deal of energy and high investment in particular in order to achieve very high temperatures that can exceed 800 °C. These processes, in addition to being very expensive to build and to operate, also generate substantial quantities of green house causing carbon dioxide. Furthermore, the "carbon atom efficiency" of such processes is undesirably low.

In addition, International Patent Application WO 98/02244 discloses a process for converting alkanes into their higher and lower homologues. According to the process, one or more starting alkanes are reacted on a metal catalyst suitable for alkane metathesis and chosen in particular from metal hydrides and organometallic compounds fixed to a solid support. However, the process relates neither to the conversion of natural gas nor to that of methane, but to that of alkanes having at least 2 carbon atoms. Various conversions are shown as examples, in particular those of ethane, propane, butane or isobutane into their higher and lower homologues.

International Patent Applications WO 01/04077 and WO 03/066552 disclose processes for preparing alkanes by reacting methane with at least one other starting alkane in the presence of an alkane metathesis metal catalyst capable of producing, in contact with alkane, reactions involving the splitting and recombining of carbon-carbon and/or carbon-hydrogen and/or carbon-metal bonds, in particular a metal catalyst chosen from the catalysts mentioned in International Patent Application WO 98/02244. Said reaction, known under the term "methane-olysis" permits the preparation of at least one or two alkanes having a number of carbon atoms less than or equal to that of the initial alkane and at least equal to 2. Various methane-olysis reactions are shown as examples, in particular by reacting methane with ethane, propane or n-butane. It appears that methane-olysis of ethane leads to formation of methane and ethane, that is to say to formation of the starting products of the reaction. In addition, it is shown that methane-olysis of propane leads to formation of ethane, and that of n-butane permits ethane and propane to be prepared. However, the methane-olysis process leads to the production of alkanes having a number of carbon atoms that is not higher than that of the starting alkane.

International Patent Application WO 03/104171 discloses a process for converting methane into ethane by contacting methane with an alkane metathesis metal catalyst chosen in particular from catalysts mentioned in International Patent Application WO 98/02244. The process employs a methane coupling reaction, also called a methane homologation reaction. It is stated that the process forms ethane with a very high selectivity by weight compared with all the carbon-containing products prepared, for example a selectivity by weight that can reach 99 % or even higher. In addition, it is suggested that the ethane thus prepared can then be used in other processes for upgrading of the ethane, such as dehydrogenation, catalytic cracking or thermal cracking of the ethane, in order to prepare more particularly olefins, e.g. ethylene. International Patent Application WO 03/ 104171 does not envisage a process for the production of higher alkanes beyond ethane, in particular higher alkanes (C₃₊), preferably (C₄₊).

International Patent Application WO 2006/060692 discloses supported metal clusters comprising one or more metals of Groups 4, 5 and/or 6 of the Periodic Table of the Elements, and which can be used as catalysts for alkane reactions. More particularly, the supported metal clusters are capable of being used to catalyze the metathesis (also referred to as disproportionation) of alkanes, conversions of alkanes with each other to give alkane products with molecular weights different from those of the starting alkanes, and conversion of methane to higher molecular-weight alkanes. The only alkane reactions described and shown as examples relate to a self-metathesis of ethane to give propane and methane, and a conversion of methane and n-butane (i.e. a methane-olysis of n-butane) to give propane and ethane. However, the Application neither discloses, nor suggests a process for converting natural gas into higher alkanes (C₃₊), preferably (C₄₊). Indeed, it is shown that methane reacting with other starting alkane (e.g. n-butane) leads to formation of final alkanes (e.g. propane and ethane) with molecular weight lower than that of the starting alkane.

Thus, in appears that none of the above-mentioned patent applications proposes a process for converting natural gas into higher alkanes having at least 3 carbon atoms (C₃₊), preferably at least 4 carbon atoms (C₄₊), and capable more particularly of being easily transported in liquid form, e.g. under standard temperature and pressure conditions or conditions close to the latter.

A process has now been found for converting natural gas into higher alkanes (C₃₊), preferably (C₄₊), and capable in particular of being easily transportable in liquid form, e.g. under standard temperature and pressure conditions or conditions close to the latter. The process has the advantage of being able to produce the higher alkanes (C₃₊), preferably (C₄₊), having a high degree of purity and in particular being free of alkenes, alkynes, aromatic compounds, carbon monoxide, carbon dioxide, sulfur- and/or nitrogen-containing products. It also has the advantage of being able to prepare said higher alkanes (C₃₊), preferably (C₄₊), with a narrow distribution of the number of carbon atoms, in particular ranging for example from 3 to 15 or preferably from 4 to 15, particularly from 3 to 13 or preferably from 4 to 13, more particularly from 3 to 10 or preferably from 4 to 10, in particular from 3 to 8 or preferably from 4 to 8 carbon atoms. The present invention has, in addition, the advantage of proposing a process by a non-oxidative route, of being simple and direct, and consequently of being easily realisable and inexpensive, so that it can be used directly on the natural gas deposit sites. Furthermore, one of the most important advantages of the process of the present invention relates to its potential for an extremely high "carbon atom efficiency" which can approach 95% or higher for converting natural gas into easily transportable mixture of higher alkanes.

More particularly, the present invention relates to a process for converting natural gas into higher alkanes having at least 3 carbon atoms (C₃₊), preferably at least 4 carbon atoms (C₄₊), characterised in that it comprises the following stages:
- a stage (1) comprising contacting natural gas with a metal catalyst (C1) capable of producing, in contact with alkane, reactions involving the splitting and recombining of carbon-carbon and/or carbon/hydrogen and/or carbon metal bonds, so as to form a mixture (M1) comprising ethane and hydrogen,
- optionally a stage (2) comprising separating ethane from the mixture (M1), so as to isolate the ethane,
- a stage (3) for ethane metathesis, comprising contacting the ethane isolated in stage (2) or the mixture (M1) obtained in stage (1) with a metal catalyst (C2) capable of producing, in contact with alkane, reactions involving the splitting and recombining of carbon-carbon and/or carbon-hydrogen and/or carbon-metal bonds, said catalyst being identical to or different from the catalyst (C1), so as to form a mixture (M2) comprising methane and higher alkanes (C₃₊), preferably (C₄₊), and
- a stage (4) comprising separating the higher alkanes (C₃₊), preferably (C₄₊), from the mixture (M2), so as to isolate said higher alkanes.

The natural gas used in the process can be any natural gas, such as that exploited directly on a deposit site. It is generally formed of a mixture of alkanes comprising mainly methane and, in weaker proportions, C₂ to C₆ alkanes, e.g. ethane, propane, butanes, pentanes and hexanes, for example with the following percentages expressed in mole:
- methane from 75 to 99%, preferably from 80 to 98%, particularly from 85 to 97%,
- ethane from 1 to 15%, preferably from 1 to 10%, particularly from 1 to 7%,
- propane from 0.1 to 10%, preferably from 0.1 to 5%, particularly from 0.1 to 3%,
- butanes (n-butane and isobutene) from 0 to 3%, preferably from 0.01 to 2%, particularly from 0.01 to 1%,
- pentanes (n-pentane and isopentane) from 0 to 2%, preferably from 0.01 to 1%, and
- hexane(s) from 0 to 1%, preferably from 0.01 to 0.1%.

Sometimes, natural gas may also comprise traces of heptane(s) and/or higher alkanes.

Natural gas can comprise nitrogen (N₂) and one or more poisons capable more particularly of deactivating metal catalysts. Thus, prior to stage (1), it may be advantageous to subject the natural gas to a purification stage for removing poison(s) capable of deactivating the metal catalyst(s) (C1) and/or (C2) employed in the present process, and optionally nitrogen (N₂). The purification stage can comprise more particularly removing one or more poisons chosen from water, carbon dioxide and sulfur compounds especially selected from hydrogen sulfide, carbonyl sulfide and mercaptans, and optionally nitrogen (N₂). Any known method can be used for removing nitrogen (N₂) and these poisons.

Several natural gas deposits comprise substantial concentrations of nitrogen (N₂) as impurity. Since nitrogen (N₂) is not anticipated to harm the catalysts (C1) and/or (C2), it might not be necessary to remove the nitrogen (N₂) from the natural gas prior to contacting these catalysts, in particular if the concentration of nitrogen is small, e.g. lower than 1% by volume of the total volume of the gas. However, it is advisable to remove nitrogen (N₂) from the natural gas before contacting the natural gas with these catalysts.

Thus, water can be removed by subjecting natural gas to a dehydration process, preferably chosen from cryogenic dehydration, dehydration by absorption and adsorptive dehydration. A cryogenic dehydration generally comprises cooling the wet natural gas until the components to be removed by condensation or formation of hydrates. Methanol, glycol or a paraffin solvent can be used during such a cryogenic dehydration. The reduction in temperature can be achieved e.g. by Joule-Thomson expansion.

Dehydration by absorption generally comprises a glycol absorption, wherein glycol such as mono-, di- or preferably triethylene glycol exhibits a high absorption capacity for water.

Adsorptive dehydration generally comprises contacting natural gas with any adsorbent suitable for retaining water, preferably chosen from molecular sieves, silica gels and Na₂O-containing silica.

Sulfur compounds and carbon dioxide can be removed from natural gas by any known process, preferably chosen from physical absorption processes, chemical absorption processes, physical-chemical absorption processes, liquid oxidation processes, adsorption processes and membrane processes.

Generally, absorption processes comprise a gas scrubbing wherein the sour natural gas components are reversibly bound to a solvent by chemical and/or physical absorption. In regeneration step, the components are then desorbed unchanged and the solvent can be recycled to the scrubber. In physical absorption processes, carbon dioxide and hydrogen sulfide are generally physically dissolved in a solvent preferably chosen from N-methylpyrrolidone, a mixture of poly(ethylene glycol dimethyl ether), poly(ethylene glycol methyl isopropyl ether) and propylene carbonate. Chemical absorption processes generally comprise an alkanolamine scrubbing wherein aqueous solutions of monoethanolamine, diethanolamine, diisopropylamine, diglycolamine or methyldiethanolamine can be used as absorbents. Physical-chemical absorption processes generally comprise using a combination of solvents described previously in physical absorption and chemical absorption processes.

Liquid oxidation processes generally comprise the following steps: (i) absorption of hydrogen sulfide in an alkaline solution, e.g. a solution of NaHCO₃-Na₂CO₃, (ii) oxidation of dissolved hydrogen sulfide ions preferably by vanadates or iron(III) aminopolycarboxylic acid chelates, to elemental sulfur with simultaneous reduction of a chemical oxygen carrier, e.g. anthraquinonedisulfonic acids, (iii) reoxidation of the active component with air, and (iv) separation of elemental sulfur by filtration, centrifugation, flotation or sedimentation.

Adsorption processes for removal of hydrogen sulfide and carbon dioxide generally comprise contacting natural gas with any known adsorbent preferably chosen from activated charcoal, iron oxide, zinc oxide and zeolitic molecular sieves.

Membrane processes can be used simultaneously for separation of carbon dioxide and methane, removal of carbon dioxide and hydrogen sulfide, dehydration and recovery of C₂₊ alkanes, preferably C₂ to C₆ alkanes or higher alkanes. Generally, membrane processes comprise the following steps: (i) absorption from the gas phase into the membrane matrix, (ii) diffusion through the membrane, and (iii) desorption out the membrane into the gas phase. Membranes generally are polymer membranes preferably chosen from asymmetric cellulose acetate or triacetate, composite layers silicone/polysulfone, composite layers polyetherimide and composite layers silicone/polycarbonate. Any known membrane process may be used to remove the nitrogen (N₂) from the natural gas. Some membranes are capable of separating more than one component, and therefore depending on the composition of the natural gas that is being processed, appropriate separation methods have to be chosen.

Stage (1) of the process comprises contacting natural gas with a metal catalyst (C1) capable of producing, in contact with alkane, reactions involving the splitting and recombining of carbon-carbon and/or carbon-hydrogen and/or carbon-metal bonds.

The catalyst (C1) can be chosen from metal catalysts which are capable of alkane metathesis. It can be chosen in particular from supported metal clusters and preferably from metal hydrides, organometallic compounds and organometallic hydrides, in particular supported on, and more specifically grafted onto, a solid support.

The catalyst (C1) can be chosen in particular from supported metal clusters comprising one or more metals of Groups 4, 5 and/or 6 of the Periodic Table of the Elements, preferably tantalum. The Table (here and hereafter) is that proposed by IUPAC in 1991 and, for example, published by CRC Press, Inc. (USA) in « CRC Handbook of Chemistry and Physics », 76th Edition (1995-1996), by David R. Lide. The supported metal clusters generally comprise a solid support and a metal cluster, and preferably comprise bonding between the metals of the metal cluster, and also bonding between the metal cluster and the solid support. Thus, the metal atom of the metal cluster can be bonded to at least one other metal atom of the metal cluster, preferably to six or fewer metal atoms of the metal cluster. The metal atom of the metal cluster can be bonded in addition to at least one hydrocarbon radical and/or to at least one hydrogen atom. The solid support can be chosen from a metal oxide, a refractory oxide, a molecular sieve, a zeolite, a metal phosphate and a material comprising a metal and oxygen, preferably silica. More particularly, the supported metal cluster can comprise tantalum (as a metal cluster) and silica (as a solid support), preferably bonding between tantalum and tantalum, and also bonding between tantalum and silica, in particular bonding between tantalum and oxygen on the silica surface. The catalyst (C1) can be chosen from the supported metal clusters described in International Patent Application WO 2006/060692, and prepared according to any of the methods described in said Application.

The catalyst (C1) is preferably chosen from metal hydrides, organometallic compounds and organometallic hydrides, particularly supported on, more particularly grafted onto, a solid support. It can comprise at least one metal chosen from lanthanides, actinides and metals of Groups 2 to 12, preferably transition metals of Groups 3 to 12, more particularly of Groups 3 to 10 of the Periodic Table of the Elements. In particular, the metal of the catalyst can be chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium. It can be chosen preferably from yttrium, titanium, zirconium, hafnium, vanadium, niobium, tantalum, chromium, molybdenum, tungsten, rhenium, ruthenium and platinum, and more especially from niobium, tantalum, molybdenum, tungsten and rhenium.

The catalyst (C1) can be chosen from metal hydrides, and preferably from organometallic compounds and organometallic hydrides, comprising in particular at least one hydrocarbon radical (R), either saturated or unsaturated, linear or branched, having preferably from 1 to 20, more particularly from 1 to 14 carbon atoms. The hydrocarbon radicals (R) can be chosen from alkyl radicals, in particular either saturated or unsaturated, linear or branched, aliphatic or alicyclic, for example alkyl, alkylidene or alkylidyne radicals, particularly from C₁ to C₁₀, from aryl radicals, in particular from C₆ to C₁₂, and from aralkyl, aralkylidene or aralkylidyne radicals, in particular from C₇ to C₁₄. Thus, the metal atom of the catalyst can preferably be bonded to at least one hydrogen atom and/or to at least one hydrocarbon radical (R), in particular by a single, double or triple carbon-metal bond.

The catalyst (C1) preferably selected from metal hydrides, organometallic compounds and organometallic hydrides, can comprise one or more ligands, such as "ancillary" ligands, preferably comprising at least one oxygen atom and/or at least one nitrogen atom, and chosen more particularly from oxo, alkoxo, aryloxo, aralkyloxo, nitrido, imido and amido ligands. Generally, by oxo, alkoxo, aryloxo, aralkyloxo, nitrido, imido and amido ligands are respectively meant:
- a divalent oxo radical of general formula: = O
- a monovalent alkoxo, aryloxo or aralkyloxo radical of general formula: - OR'
- a trivalent nitrido radical of general formula: = N
- a divalent imido radical of general formula: = NR" , and
- a monovalent amido radical of general formula : - NR¹R² , general formulae in which O represents an oxygen atom, N represents a nitrogen atom, R' represents a hydrogen atom or a monovalent hydrocarbon radical selected respectively from alkyl, aryl and aralkyl radicals, R" represents a hydrogen atom or a monovalent hydrocarbon radical, and R¹ and R², being identical or different, represent a hydrogen atom or a monovalent hydrocarbon radical. More particularly, R', R", R¹ and R² can be a monovalent hydrocarbon radical, either saturated or unsaturated, linear or branched, comprising from 1 to 20, preferably from 1 to 14 carbon atoms, and particularly chosen from alkyl radicals, preferably from C₁ to C₁₀, aryl radicals, preferably from C₆ to C₁₂, and aralkyl radicals, preferably from C₇ to C₁₄.

Thus, when the catalyst (C1), preferably selected from metal hydrides, organometallic compounds and organometallic hydrides, comprises at least of the above-mentioned ligands, the metal of the catalyst is not only bonded to at least one hydrogen atom and/or to at least one hydrocarbon radical (R), but also to at least one of these ligands, e.g. to the O atom of the oxo radical by a double bond, or the O atom of the alkoxo, aryloxo or aralkyloxo radical (OR') by a single bond, or to the N atom of the nitrido radical by a triple bond, or to the N atom of the imido radical (NR") by a double bond, or to the N atom of the amido radical (NR¹R²) by a single bond. Generally, the presence of the oxo, alkoxo, aryloxo, aralkyoxo, nitrido, imido and/or amido ligands in the catalyst (C1) can favourably influence the behaviour of the catalyst in the contacting of stage (1).

In addition, the catalyst (C1) chosen from metal hydrides, organometallic compounds and organometallic hydrides is preferably supported on, or more particularly grafted onto, a solid support that can be chosen from metal or refractory oxides, sulfides, carbides, nitrides and salts, and from carbon, mesoporous materials, organic/inorganic hybrid materials, metals and molecular sieves. The solid support is in particular chosen from silicas, aluminas, silica-aluminas and aluminium silicates, either simple or modified by other metals, and from zeolites, clays, titanium oxide, cerium oxide, magnesium oxide, niobium oxide, tantalum oxide and zirconium oxide. It can be chosen, preferably, from silicas, aluminas, silica-aluminas and aluminium silicates, either simple or modified by other metals. The solid support can have a specific surface area (B.E.T.) measured according to the standard ISO 9277 (1995) which is chosen in a range of from 0.1 to 5000 m²/g, preferably from 1 to 3000 m²/g, particularly from 1 to 1000 m²/g. It is preferred to use a metal catalyst (C1) grafted onto a solid support and in which the metal atom can be bonded to at least one hydrogen atom and/or to at least one hydrocarbon radical (R), and also to at least one of the atoms of the support, in particular to at least one atom of oxygen, sulfur, carbon or nitrogen of the support, when the support is chosen in particular from metal or refractory oxides, sulfides, carbides, nitrides and salts. The catalyst (C1) can be preferably a metal hydride or an organometallic hydride grafted onto a solid support, more particularly chosen from niobium hydrides or organometallic niobium hydrides grafted onto silica, tantalum hydrides or organometallic tantalum hydrides grafted onto silica, molybdenum hydrides or organometallic molybdenum hydrides grafted onto silica, tungsten hydrides or organometallic tungsten hydrides grafted onto alumina, and rhenium hydrides or organometallic rhenium hydrides grafted onto silica.

The catalyst (C1) selected from metal hydrides, organometallic compounds and organometallic hydrides can be in particular chosen from the metal catalysts described in the International Patent Applications WO 98/02244, WO 03/061823 and WO 2004/089541, and prepared according to any of the methods described in said Applications.

It is observed that stage (1) leads to formation of a mixture (M1) comprising ethane and hydrogen, and optionally one or more unreacted natural gas components, more particularly unreacted methane and one or more unreacted C₃₊ alkanes of the natural gas, such as C₃ to C₆ alkanes (hereafter referred as (C₃₋₆) alkanes), e.g. unreacted propane, butanes, pentanes and hexanes. It should be noted that the concentration of (C₃₋₆) alkanes in mixture (M1) can be substantially less than the concentration of the same in the natural gas before starting stage (1).

Without wishing to be bound by any theory relating to reaction mechanisms, it is thought that stage (1) can involve a plurality of simultaneous reactions leading finally to a mixture (M1) comprising in particular ethane and hydrogen. Because of the preponderance of methane compared to the other alkanes of the natural gas involved in this stage, stage (1) can involve a non-oxidative methane coupling reaction (i.e. a methane homologation reaction) leading to the production of ethane and hydrogen, in particular according to the following equation (1):

2 CH₄ → C₂H₆ + H₂ (1)

It is also observed that stage (1) can also involve methane-olysis reactions between the methane and each of the C₃₊ alkanes of the natural gas, in particular the (C₃₋₆) alkanes, e.g. propane, butanes, pentanes and hexanes, and more particularly:
- a methane-olysis reaction of propane, in particular according to the following equation (2):

   CH₄ + C₃H₈ → 2 C₂H₆ (2)
- methane-olysis reactions of butanes, in particular according to the following equations (3), (2) and (4):

   CH₄ + C₄H₁₀ → C₂H₆ + C₃H₈ (3)

   CH₄ + C₃H₈ → 2C₂H₆ (2)

   that is to say overall: 2 CH₄ + C₄H₁₀ → 3 C₂H₆ (3) + (2) = (4)
- methane-olysis reactions of pentanes, in particular according to the following equations (5), (4) and (6):

   CH₄ + C₅H₁₂ → C₂H₆ + C₄H₁₀ (5)

   2CH₄ + C₄H₁₀ → 3 C₂H₆ (4)

   that is to say overall: 3 CH₄ + C₅H₁₂ → 4 C₂H₆ (5) + (4) = (6)
- and methane-olysis reactions of hexanes, in particular according to the following equations (7), (6) and (8):

   CH₄ + C₆H₁₄ → C₂H₆ + C₅H₁₂ (7)

   3 CH₄ + C₅H₁₂ → 4 C₂H₆ (6)

   that is to say overall: 4 CH₄ + C₆H₁₄ → 5 C₂11₆ (7) + (6) = (8)

Thus, the methane-olysis reactions of propane, butanes, pentanes and hexanes can be summarised overall according to the following equation (9), which is the sum of equations (2), (4) and (6):
10 CH₄ + C₃H₈ + C₄H₁₀ + C₅H₁₂ + C₆H₁₄ → 14 C₂H₆ (2) + (4) + (6) + (8) =(9)

It is observed that in stage (1), the contacting of natural gas with the catalyst (C1) makes it possible to produce a mixture (M1) finally comprising ethane and hydrogen, and optionally unreacted methane and one or more unreacted C₃₊ alkanes of the natural gas such as the (C₃₋₆) alkanes, e.g. propane, butanes, pentanes and hexanes existing at very low concentrations. Under the operating conditions of stage (1), the mixture (M1) is generally obtained in gaseous form or in mixed gaseous/liquid form.

Stage (1) can be performed in conditions suited to promoting the development of the (non-oxidative) methane coupling reaction and of the methane-olysis reactions, e.g. methane-olysis reactions of propane, butanes, pentanes and hexanes, towards an optimum production of ethane. Thus, it can be performed at a temperature chosen in a range of from 50 to 700 °C, preferably from 70 to 650 °C, more particularly from 100 to 600 °C. It can also be performed under an absolute total pressure chosen in a range of from 0.1 to 100 MPa, preferably from 0.1 to 50 MPa, more particularly from 0.1 to 30 MPa. Stage (1) can be performed advantageously under a partial pressure of methane chosen in a range of from 0.1 to 50 MPa, preferably from 0.1 to 30 MPa, more particularly from 0.2 to 20 MPa, in particular from 0.5 to 10 MPa. It can also be performed in the presence of at least one inert agent, either liquid or gaseous, in particular an inert gas chosen from nitrogen, argon and helium.

Stage (1) can be performed in various ways, either intermittently, or discontinuously, or preferably continuously, in a reaction zone (Z1). It is possible, for example, to add the natural gas to the catalyst (C1), or to add the catalyst (C1) to the natural gas, or else to add the natural gas and the catalyst (C1) simultaneously into the reaction zone (Z 1).

The reaction zone (Z1) can be either separate and distinct from a fractionation zone (F1) wherein stage (2) is performed, e.g. in at least one reactor, or arranged in a part of said fractionation zone (F1), e.g. in distillation column reactor, as described in American Patent US 4,242,530.

In addition, the reaction zone (Z1) can comprise at least one reactor chosen from static reactors, recycling reactors and dynamic reactors. For example, stage (1) can be performed in a static reactor containing fixed quantities of natural gas and of catalyst (C 1). Stage (1) can also be performed in a recycling reactor into which at least one or more component(s) of the natural gas and/or of the mixture (M1) (for example methane, propane, butanes, pentanes and hexanes) can be recycled preferably continuously. Stage (1) can also be performed in a dynamic reactor containing the catalyst (C1), particularly in the form of solid particles, through which the natural gas can pass or circulate, preferably continuously.

In practice, stage (1) can be performed in a reaction zone (Z1) comprising at least one reactor chosen from tubular (or multi-tubular) reactors, distillation column reactors, slurry reactors, fluidised bed reactors, mechanically agitated bed reactors, fluidised and mechanically agitated bed reactors, fixed bed reactors, moving bed reactors and circulating bed reactors. The catalyst (C1) is generally solid, and can optionally be mixed with an inert solid agent chosen in particular from silicas, aluminas, silica-aluminas and aluminium silicates, either simple or modified by other metals. The catalyst (C1) can be arranged inside the tube(s) of a tubular (or multi-tubular) reactor. It can also be arranged inside a distillation column reactor, wherein the catalyst is preferably a component of a distillation system functioning as both a catalyst and a distillation packing, i.e. a packing for a distillation column having both a distillation function (as in stage (2)) and a catalytic function (as in stage (1)): for example, rings, saddles, balls, irregular shapes, granulates, sheets, tubes, spirals, packed in bags, as described in American Patent US 4,242,530. The catalyst (C1) can also form the bed of a fluidised and/or mechanically agitated bed, a fixed bed, a moving bed or a circulating bed of said reactors. The natural gas can be introduced into one of said reactors preferably continuously, and generally can pass or circulate preferably continuously in the form of a gaseous stream into the tube(s), or through the bed or the distillation packing of said reactors. In order to promote the development of the reactions towards an optimum production of ethane, stage (1) can be advantageously performed by withdrawing at least one component of the mixture (M1) such as ethane out of the reaction zone (Z1), preferably continuously.

Thus, for example, stage (1) can be performed:
(i) by continuously introducing natural gas into the reaction zone (Z1) containing the catalyst (C1), so as to form continuously the mixture (M1) comprising ethane, hydrogen and optionally one or more unreacted component(s) of the natural gas, more particularly unreacted methane and one or more unreacted C₃₊ alkanes of the natural gas such as the (C₃₋₆) alkanes, e.g. propane, butanes, pentanes and hexanes, and
(ii) by continuously withdrawing at least a part of the mixture (M1) out of the reaction zone (Z1), so as to submit said part of the mixture (M1) optionally to stage (2) for separating ethane from the mixture (M1) and isolating ethane from the rest of the mixture, or directly to stage (3), and preferably to return said rest of the mixture, preferably just the unreacted methane into the reaction zone (Z1), in particular so as to continue the contacting of stage (1).

The process then optionally comprises a stage (2) comprising separating ethane from the mixture (M1), so as to isolate it. Stage (2) may also comprise separating from the mixture (M1), ethane together with one or more unreacted C₃₊ alkanes of the natural gas, such as the (C₃₋₆) alkanes, e.g. propane, butanes, pentanes and hexanes, so as to isolate the ethane in admixture with said unreacted C₃₊ alkanes, and in particular to use the ethane in such an admixture subsequently in stage (3).

Stage (2) can be performed in various ways, either intermittently, or discontinuously, or preferably continuously, in a fractionation zone (F1). It can comprise one or more methods of fractionation of the mixture (M1), in particular chosen from any known methods or combinations of method for fractionation of such a mixture, preferably chosen from the following six types of fractionation:
- fractionation by change of physical state, preferably by of gaseous/liquid phase of the mixture, in particular by distillation or by condensation (or liquefaction), more particularly by partial condensation (or partial liquefaction), e.g. in distillation/condensation column or tower,
- fractionation by molecular filtration, preferably by means of semi-permeable and selective membrane,
- fractionation by adsorption, preferably by means of molecular sieve or any other adsorbent,
- fractionation by absorption, preferably by means of absorbing oil,
- fractionation by cryogenic expansion, preferably by means of expansion turbine, and
- fractionation by compression, preferably by means of gas compressor, so as in particular to separate and to isolate ethane preferably in admixture with one or more unreacted C₃₊ alkanes of the natural gas such as (C₃₋₆) alkanes, e.g. propane, butanes, pentanes and hexanes, and optionally hydrogen and unreacted methane.

Any type of fractionation that is useful in general for separating ethane preferably in admixture with one or more C₃₊ alkanes of the natural gas such as the (C₃₋₆) alkanes, from hydrogen and optionally methane, can be utilized in stage (2).

Stage (2) thus makes it possible firstly to separate ethane from the mixture (M1) and to isolate the ethane preferably in admixture with one or more unreacted C₃₊ alkanes of the natural gas such as the (C₃₋₆) alkanes. Furthermore, it can permit hydrogen to be separated and isolated from the mixture (M1) and preferably to be used for any applications or stages directly used or not in the present process. It can also permit the unreacted methane to be separated and isolated from the mixture (M1) and preferably to be recycled into stage (1).

Stage (2) can be performed in a fractionation zone (F1) which is either separate and distinct from the reaction zone (Z1) wherein stage (1) is performed, e.g. in at least one distillation/condensation column or tower, or arranged in a part of said reaction zone (Z1), e.g. in a distillation column reactor, such as described in American Patent US 4,242,530.

Thus, a distillation column reactor can be advantageously used both for stage (1) and stage (2), and can contain the catalyst (C1). More particularly, the catalyst (C1) can be arranged inside the distillation column reactor, as a component of a distillation system functioning as both a catalyst and a distillation packing. Thus, stages (1) and (2) can be simultaneously performed in a distillation column reactor comprising both a reaction zone and a fractionation zone. In the reaction zone, the contacting of the natural gas with the catalyst (C1) can be performed as in stage (1), so as to form the mixture (M1) preferably in a gaseous/liquid form. Simultaneously in the fractionation zone, the separation of ethane optionally in admixture with one or more unreacted C₃₊ alkanes of the natural gas such as the (C₃₋₆) alkanes, from the mixture (M1) can be performed as in stage (2), in particular by distillation or by condensation, more particularly by partial condensation, so as to isolate the ethane preferably in admixture with said C₃₊ alkanes.

More generally, a fractionation of the mixture (M1) can be performed by change of the physical state, preferably of the gaseous/liquid phase of the mixture, in particular by distillation or by condensation, more particularly by partial condensation, e.g. in at least one distillation/condensation column or tower, or in a distillation column reactor, in particular by refrigeration or cryogenic processes. Preferably it can be performed by at least one cooling of the gaseous mixture (M1) to at least one temperature at which at least one component of the mixture condenses and the rest of the mixture remains in gaseous form, so as to condense, to separate and to isolate said component(s) from said rest of the mixture.

Generally, a fractionation of the mixture (M1) can also be performed by molecular filtration, in particular by passing the gaseous mixture (M1) through at least one semi-permeable and selective membrane, so as to arrest and to isolate at least one component of the mixture and to allow the rest of the mixture to pass through.

Generally, a fractionation of the mixture (M1) can also be performed by adsorption, in particular by passing the gaseous mixture (M1) onto at least one molecular sieve or any other adsorbent, so as to retain at least one component of the mixture and to allow the rest of the mixture to pass through, and by submitting the component(s) thus retained to at least one desorption step, preferably by the TSA method ("Temperature Swing Adsorption") or the PSA method ("Pressure Swing Adsorption"), so as to isolate said component(s).

Generally, a fractionation of the mixture (M1) can also be performed by absorption, preferably by contacting the gaseous mixture (M1) with an absorbing oil, e.g. in an absorption tower, so as to soak up at least one component of the mixture, to form an oil/component mixture and to allow the rest of the gaseous mixture to be free and not absorbed, then by subjecting said oil/component mixture, e.g. in a recovery tower, to a temperature above the boiling point of the component, but below that of the oil, so as to separate and to isolate said component from the oil.

Generally, a fractionation of the mixture (M1) can also be performed by cryogenic expansion, preferably by dropping the temperature of the gaseous mixture (M1) to a temperature so as to condense at least one component of the mixture while maintaining the rest of the mixture in gaseous form, with the help of an expansion turbine which rapidly expands the gaseous mixture and causes a significant drop of the temperature.

Generally, a fractionation of the mixture (M1) can also be performed by compression, in particular by compressing the gaseous mixture (M1) e.g. by means of a gas compressor, so as to condense at least one component of the mixture, to separate and to isolate said component, and to allow the rest of the mixture to be maintained in gaseous form.

For example, a fractionation of the gaseous mixture (M1) can be performed by change of the physical state, preferably of the gaseous/liquid phase of the mixture, e.g. in at least one distillation/condensation column or tower, or in a distillation column reactor, in particular by cooling the gaseous mixture (M1) to a temperature at which ethane and preferably one or more unreacted C₃₊ alkanes of the natural gas such as the (C₃₋₆) alkanes e.g. propane, butanes, pentanes and hexanes condense, so as to condense the ethane and preferably said C₃₊ alkanes, and to separate and to isolate it (or them) in liquid form from the rest of the gaseous mixture comprising hydrogen and optionally unreacted methane.

For example, a fractionation of the gaseous mixture (M1) can also be performed by molecular filtration of the mixture, preferably by passing the gaseous mixture (M1) through at least one semi-permeable and selective membrane, so as to arrest and to isolate ethane and preferably one or more unreacted C₃₊ alkanes of the natural gas such as the (C₃₋₆) alkanes, e.g. propane, butanes, pentanes and hexanes, and to allow the rest of the gaseous mixture comprising hydrogen and optionally unreacted methane to pass through.

The semi-permeable and selective membranes can be chosen from those suitable for arresting and separating ethane from hydrogen and optionally methane, preferably chosen from zeolite membranes, porous alumina membranes, ceramic membranes, flowing liquid membranes and supported liquid membranes.

For example, a fractionation of the gaseous mixture (M1) can also be performed by adsorption, preferably by passing the gaseous mixture (M1) onto at least one molecular sieve or any other adsorbent, so as to retain ethane and preferably one or more C₃₊ alkanes of the natural gas such as the (C₃₋₆) alkanes, e.g. propane, butanes, pentanes and hexanes, and to allow the rest of the gaseous mixture comprising hydrogen and optionally unreacted methane to pass through, and by submitting the ethane and preferably said C₃₊ alkanes thus retained to at least one desorption step, in particular by the TSA or the PSA method, so as to isolate the ethane preferably in admixture with said C₃₊ alkanes.

The adsorbents can be chosen from those suitable for retaining and separating ethane from hydrogen and optionally methane, preferably chosen from molecular sieves, silica gels, activated charcoals and activated carbons.

For example, a fractionation of the gaseous mixture (M1) can also be performed by absorption, preferably by contacting the gaseous mixture (M1) with an absorbing oil, e.g. in an absorption tower, so as to soak up ethane and preferably one or more unreacted C₃₊ alkanes of the natural gas such as the (C₃₋₆) alkanes, e.g. propane, butanes, pentanes and hexanes, and to form an oil/ethanelpreferably C₃₊ alkane mixture, and then preferably by subjecting said mixture, e.g. in a recovery tower, to a temperature above the boiling point of ethane and preferably of C₃₊ alkanes, but below that of the oil, so as to separate and to isolate the ethane preferably with said C₃₊ alkanes from the oil and from the rest of the gaseous mixture comprising hydrogen and optionally unreacted methane.

For example, a fractionation of the gaseous mixture (M1) can also be performed by cryogenic expansion, preferably by dropping the temperature of the gaseous mixture (M1) in particular by use of external refrigerants to cool the gaseous mixture and then by means of an expansion turbine which rapidly expands the chilled gaseous mixture and causes the temperature to drop significantly, so as to condense ethane and preferably one or more unreacted C₃₊ alkanes of the natural gas such as the (C₃₋₆) alkanes e.g. propane, butanes, pentanes and hexanes, to separate and to isolate it (or them) in liquid form from the rest of the gaseous mixture comprising hydrogen and optionally unreacted methane.

For example, a fractionation of the gaseous mixture (M1) can also be performed by compression, preferably by compressing the gaseous mixture (M1), e.g. by means of a gas compressor, so as to condense ethane and preferably one or more unreacted C₃₊ alkanes of the natural gas such as the (C₃₋₆) alkanes, e.g. propane, butanes, pentanes and hexanes, to separate and to isolate it (or them) in liquid form from the rest of the gaseous mixture comprising hydrogen and optionally unreacted methane.

Stage (2) can also be performed advantageously by a double (or multiple) fractionation of the mixture (M1), comprising a combination of two (or more) successive fractionations of an identical or different type, preferably chosen from the six above-mentioned types of fractionation, so as in particular to separate and to isolate (a) ethane preferably in admixture with one or more unreacted C₃₊ alkanes of the natural gas such as the (C₃₋₆) alkanes, e.g. propane, butanes, pentanes and hexanes, (b) hydrogen and (c) unreacted methane, from the mixture (M1). The unreacted methane can be advantageously separated and isolated in stage (2) and then preferably recycled into stage (1). In addition, it may also be particularly advantageous to separate and to isolate the hydrogen which is then preferably used in one or more applications or stages, distinct or not from the present process. For example, the hydrogen thus isolated can be then used as an agent for activation or regeneration of the catalyst(s) (C1) and/or (C2) employed in the process. The activation or the regeneration of the catalyst can be carried out by contacting the catalyst with the hydrogen, either in one of the stages of the process, in particular stage (3) for ethane metathesis, or in a stage distinct and separate from the process. The hydrogen can also be used in other applications distinct or not from the present process, for example as a fuel for producing thermal and/or electrical energies, in particular as a fuel in hydrogen fuel cells for producing electrical energy, such energies being preferably employed directly in the present process, or as a fuel for automobile, or as a reagent in a chemical, petrochemical or refinery plant. It is remarkable to notice that hydrogen produced and isolated by the process can generally supply all the energy needed to run said process. In addition, it can be particularly advantageous to separate and to isolate the ethane in admixture with one or more unreacted C₃₊ alkanes of the natural gas such as the (C₃₋₆) alkanes, e.g. propane, butanes, pentanes and hexanes, since the ethane thus isolated with such an admixture can be then used more efficiently in stage (3) for ethane metathesis, and can also improve very substantially the output of the process.

For example, it is possible to perform a double fractionation by subjecting the gaseous mixture (M1) comprising ethane, hydrogen, unreacted methane and one or more unreacted C₃₊ alkanes of the natural gas such as the (C₃₋₆) alkanes, e.g. propane, butanes, pentanes and hexanes, to a combination of two successive fractionations of one and the same type, namely by change of the physical state, preferably of the gaseous/liquid phase of the mixture, in particular by means of one or more distillation/ condensation columns or towers, or of a distillation column reactor, as follows:
(i) by a first cooling of the gaseous mixture (M1) to a temperature at which ethane and preferably one or more unreacted C₃₊ alkanes of the natural gas such as the (C₃₋₆) alkanes e.g. propane, butanes, pentanes and hexanes condense, so as to condense the ethane and preferably said C₃₊ alkane(s), to separate and to isolate it (or them) in liquid form from the rest of the gaseous mixture comprising unreacted methane and hydrogen, and
(ii) by a second cooling of said rest of the gaseous mixture to a temperature at which unreacted methane condenses, so as to condense the methane, to separate and to isolate it in liquid form from hydrogen which is preferably isolated in its turn in gaseous form.

For example, it is also possible to perform a double fractionation by subjecting the above-mentioned gaseous mixture (M1) to a combination of two successive fractionations of one and the same type, namely by molecular filtration:
(i) by passing the gaseous mixture (M1) through a first semi-permeable and selective membrane, so as to arrest and to isolate ethane and preferably one or more unreacted C₃₊ alkanes of the natural gas such as the (C₃₋₆) alkanes e.g. propane, butanes, pentanes and hexanes, and to allow the rest of the gaseous mixture comprising hydrogen and unreacted methane to pass through, and
(ii) by passing said rest of the gaseous mixture through a second semi-permeable and selective membrane, so as to arrest and to isolate methane, and to allow hydrogen to pass through and preferably to be isolated in its turn in gaseous form.

For example, it is also possible to perform a double fractionation by subjecting the above-mentioned gaseous mixture (M1), to a combination of two successive fractionations of one and the same type, namely by molecular adsorption:
(i) by passing the gaseous mixture (M1) onto a first molecular sieve or any other adsorbent, so as to retain ethane and preferably one or more unreacted C₃₊ alkanes of the natural gas such as the (C₃₋₆) alkanes, e.g. propane, butanes, pentanes and hexanes, and to allow the rest of the gaseous mixture comprising hydrogen and unreacted methane to pass through, and
(ii) by passing said rest of the gaseous mixture onto a second molecular sieve or any other adsorbent, so as to retain methane, and to allow hydrogen to pass through and preferably to be isolated in gaseous form,
(iii) by respectively submitting ethane preferably in admixture with said C₃₊ alkanes and unreacted methane thus retained to desorption steps, preferably by the TSA or PSA method, so as to isolate the ethane preferably in admixture with said C₃₊ alkanes and the unreacted methane.

For example, it is also possible to perform a double fractionation by subjecting the above-mentioned gaseous mixture (M1) to a combination of two successive fractionations of one and the same type, namely by absorption, e.g. in at least one or two absorption towers:
(i) by contacting the gaseous mixture (M1) with a first absorbing oil, so as to soak up the ethane and preferably one or more unreacted C₃₊ alkanes of the natural gas such as the (C₃₋₆) alkanes e.g. propane, butanes, pentanes and hexanes, to form an oil/ethane/preferably C₃₊ alkane mixture, and to allow the rest of the gaseous mixture comprising unreacted methane and hydrogen to be free in gaseous form,
(ii) by contacting said rest of the gaseous mixture with a second absorbing oil, so as to soak up methane, to form an oil/methane mixture, and to allow hydrogen to be free in gaseous form and preferably to be isolated, and
(iii) by subjecting said oil/ethane/preferably C₃₊ alkane mixture, e.g. in a recovery tower, to a temperature above the boiling point of ethane and preferably of said C₃₊ alkanes, but below that of the oil, so as to separate and to isolate the ethane preferably with said C₃₊ alkanes from the oil, and preferably by subjecting said oil/methane mixture, e.g. in a recovery tower, to a temperature above the boiling point of methane, but below that of the oil, so as to separate and to isolate the unreacted methane from the oil.

For example, it is also possible to perform a double fractionation by subjecting the above-mentioned gaseous mixture (M1) to a combination of two successive fractionations of one and the same type, namely by cryogenic expansion:
(i) by dropping the temperature of the gaseous mixture (M 1) by means of an expansion turbine, so as to condense ethane and preferably one or more unreacted C₃₊ alkanes of the natural gas such as the (C₃₋₆) alkanes e.g. propane, butanes, pentanes and hexanes, to separate and to isolate it (or them) in liquid form from the rest of the gaseous mixture comprising unreacted methane and hydrogen, and
(ii) by dropping the temperature of said rest of the gaseous mixture by means of an expansion turbine, so as to condense methane, to separate and to isolate it in liquid form from hydrogen which is preferably isolated in its turn in gaseous form.

For example, it is also possible to perform a double fractionation by subjecting the above-mentioned gaseous mixture (M1) to a combination of two successive fractionations of one and the same type, namely by compression:
(i) by compressing the gaseous mixture (M1), e.g. by means of a gas compressor, so as to condense ethane and preferably one or more unreacted C₃₊ alkanes of the natural gas such as the (C₃₋₆) alkanes e.g. propane, butanes, pentanes and hexanes, to separate and to isolate it (or them) in liquid form from the rest of the gaseous mixture comprising unreacted methane and hydrogen, and
(ii) by compressing said rest of the gaseous mixture, e.g. by means of a gas compressor, so as to condense methane, to separate and to isolate it in liquid form from hydrogen which is preferably isolated in its turn in gaseous form.

It is also possible to perform a double (or multiple) fractionation by subjecting the mixture (M1) comprising ethane, hydrogen, unreacted methane and one or more unreacted C₃₊ alkanes of the natural gas such as the (C₃₋₆) alkanes, e.g. propane, butanes, pentanes and hexanes, to a combination of two successive fractionations of a different type, preferably chosen from the six above-mentioned types of fractionation.

More particularly, a double fractioning of the above-mentioned mixture (M1) can be performed by combining two successive fractionations of a different type, namely:
- the one by change of the physical state, preferably of the gaseous/liquid phase of the mixture, in particular by distillation or by condensation, and the other by molecular filtration, preferably by means of a semi-permeable and selective membrane, or
- the one by change of the physical state, preferably of the gaseous/liquid phase of the mixture, in particular by distillation or by condensation, and other by adsorption, in particular by means of a molecular sieve or any other adsorbent, or
- the one by change of the physical state, preferably of the gaseous/liquid phase of the mixture, in particular by distillation or by condensation, and the other by absorption, preferably by means of an absorbing oil, or
- the one by change of the physical state, preferably of the gaseous/liquid phase of the mixture, in particular by distillation or by condensation, and the other by cryogenic expansion, preferably by means of an expansion turbine, or
- the one by change of the physical state, preferably of the gaseous/liquid phase of the mixture, in particular by distillation or by condensation, and the other by compression, preferably by means of a gas compressor, or
- the one by molecular filtration, preferably by means of a semi-permeable and selective membrane, and the other by adsorption, preferably by means of a molecular sieve or any other adsorbent, or
- the one by molecular filtration, preferably by means of a semi-permeable and selective membrane, and the other by absorption, preferably by means of an absorbing oil, or
- the one by molecular filtration, preferably by means of a semi-permeable and selective membrane, and the other by cryogenic expansion, preferably by means of an expansion turbine, or
- the one by molecular filtration, preferably by means of a semi-permeable and selective membrane, and the other by compression, preferably by means of a gas compressor, or
- the one by adsorption, preferably by means of a molecular sieve or any other adsorbent, and the other by absorption, preferably by means of an absorbing oil, or
- the one by adsorption, preferably by means of a molecular sieve or any other adsorbent, and the other by cryogenic expansion, preferably by means of an expansion turbine, or
- the one by adsorption, preferably by means of a molecular sieve or any other adsorbent, and the other by compression, preferably by means of a gas compressor, or
- the one by absorption, preferably by means of an absorbing oil, and the other by cryogenic expansion, preferably by means of an expansion turbine, or
- the one by absorption, preferably by means of an absorbing oil, and the other by compression, preferably by means of a gas compressor, or
- the one by cryogenic expansion, preferably by means of an expansion turbine, and the other by compression, preferably by means of a gas compressor.

Each of said combinations can be performed in a given chronological order or in a reverse order.

Thus, for example, the two successive fractionations of a different type can be performed advantageously:
(i) by cooling the gaseous mixture (M1) to a temperature at which ethane and preferably one or more unreacted C₃₊ alkanes of the natural gas such as the (C₃₋₆) alkanes e.g. propane, butanes, pentanes and hexanes condense, so as to condense the ethane and preferably said C₃₊ alkane(s), to separate and to isolate it (or them) in liquid form from the rest of the gaseous mixture comprising unreacted methane and hydrogen, and
(ii) by passing said rest of the gaseous mixture through a semi-permeable and selective membrane, so as to arrest and to isolate methane, and to allow hydrogen to pass through and preferably to be isolated in its turn in gaseous form.

For example, it is also possible to perform the two above-mentioned successive fractionations in a reverse order, that is to say:
(i) by passing the gaseous mixture (M1) through a semi-permeable and selective membrane, so as to arrest and to isolate ethane and preferably one or more unreacted C₃₊ alkanes of the natural gas such as the (C₃₋₆) alkanes, e.g. propane, butanes, pentanes and hexanes, and to allow the rest of the gaseous mixture comprising unreacted methane and hydrogen to pass through, and
(ii) by cooling said rest of the gaseous mixture to a temperature at which methane condenses, to separate and to isolate methane in liquid form from hydrogen which is preferably isolated in its turn in gaseous form.

Thus, by means of a double (or multiple) fractionation such as one of those described above, the unreacted methane thus isolated in stage (2) is preferably recycled into stage (1). In addition, the hydrogen thus isolated in stage (2) is preferably used in at least one of the above-mentioned applications or stages.

The process then comprises a stage (3) for ethane metathesis, comprising contacting ethane isolated in stage (2) or the mixture (M1) obtained in stage (1) with a metal catalyst (C2) capable of producing, in contact with alkane, reactions involving the splitting and re-combining of carbon-carbon and/or carbon-hydrogen and/or carbon-metal bonds, so as to form a mixture (M2) comprising methane and the higher alkanes having at least 3 carbon atoms (C₃₊), preferably at least 4 carbon atoms (C₄₊). In stage (3), the contacting with the catalyst (C2) can be preferably performed with ethane (or the mixture (M1)) in admixture with one or more unreacted C₃₊ alkanes of the natural gas such as the (C₃₋₆) alkanes, e.g. propane, butanes, pentanes and hexanes, thus isolated in stage (2).

The catalyst (C2) can be identical to or preferably different from the catalyst (C1) used in stage (1), and can conform more particularly to the same definitions of metal catalysts given above for the catalyst (C1). In the case when the catalyst (C2) is different from the catalyst (C1), it might be possible to use directly in stage (3) the mixture (M1) obtained in stage (1), instead of using the ethane isolated in stage (2).

The catalyst (C2) can be chosen from metal catalysts that are capable of alkane metathesis. It can be chosen in particular from supported metal clusters, and preferably from metal hydrides, organometallic compounds and organometallic hydrides, particularly supported on, more particularly grafted onto, a solid support.

The catalyst can be chosen in particular from supported metal clusters comprising one or more metals of Groups 4, 5 and/or 6 of the Periodic Table of the Elements, preferably tantalum. The supported metal clusters generally comprise a solid support and a metal cluster, and preferably comprise bonding between the metals of the metal cluster, and also bonding between the metal cluster and the solid support. Thus, the metal atom of the metal cluster can be bonded to at least one other metal atom of the metal cluster, preferably to six or fewer metal atoms of the metal cluster. The metal atom of the metal cluster can be bonded in addition to at least one hydrocarbon radical and/or to at least one hydrogen atom. The solid support can be chosen from a metal oxide, a refractory oxide, a zeolite, a metal phosphate and a material comprising a metal and oxygen, and preferably silica. More particularly, the supported metal cluster can comprise tantalum (as a metal cluster) and silica (as a solid support), preferably bonding between tantalum and tantalum, and also bonding between tantalum and silica, in particular bonding between tantalum and oxygen on the silica surface. The catalyst (C2) can be chosen from the supported metal clusters described in International Patent Application WO 2006/060692, and prepared according to any methods described in said Application.

The catalyst (C2) is preferably chosen from metal hydrides, organometallic compounds and organometallic hydrides, particularly supported on, more specifically grafted onto, a solid support. It can comprise at least one metal particularly chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium. The metal of the catalyst (C2) can be preferably chosen from yttrium, titanium, zirconium, hafnium, vanadium, niobium, tantalum, chromium, molybdenum, tungsten, rhenium, ruthenium and platinum, and more especially chosen from niobium, tantalum, molybdenum, tungsten and rhenium.

The catalytic (C2) can be chosen from metal hydrides, and preferably from organometallic compounds and organometallic hydrides, comprising more particularly at least one hydrocarbon radical (R), either saturated or unsaturated, linear or branched, preferably comprising from 1 to 20, more particularly from 1 to 14 carbon atoms. The hydrocarbon radicals (R) can be chosen from alkyl radicals, preferably either saturated or unsaturated, linear or branched, aliphatic or alicyclic, for example alkyl, alkylidene or alkylidyne radicals, more particularly from C₁ to C₁₀, from aryl radicals, more particularly from C₆ to C₁₂, and from aralkyl, aralkylidene or aralkylidyne radicals, more particularly from C₇ to C₁₄. Thus, the metal atom of the catalyst (C2) is preferably bonded to at least one hydrogen atom and/or to at least one hydrocarbon radical (R), in particular by a single, double or triple carbon-metal bond.

The catalyst (C2) preferably selected from metal hydrides, organometallic compounds and organometallic hydrides, can comprise one or more ligands, such as "ancillary" ligands, preferably comprising at least one oxygen atom and/or at least one nitrogen atom, in particular chosen from oxo, alkoxo, aryloxo, aralkyloxo, nitrido, imido and amido ligands. Generally, by oxo, alkoxo, aryloxo, aralkyloxo, nitrido, imido and amido ligands are respectively meant:
- a divalent oxo radical of general formula: = O
- a monovalent alkoxo, aryloxo or aralkyloxo radical of general formula: - OR'
- a trivalent nitrido radical of general formula: = N
- a divalent imido radical of general formula: = NR" , and
- a monovalent amido radical of general formula: - NR¹R² general formulae in which O represents an oxygen atom, N represents a nitrogen atom, R' represents a hydrogen atom or a monovalent hydrocarbon radical selected respectively from alkyl, aralkyl and aralkyl radicals, R" represents a hydrogen atom or a monovalent hydrocarbon radical, and R¹ and R², being identical or different, represent a hydrogen atom or a monovalent hydrocarbon radical. More particularly, R', R", R¹ and R² can be a monovalent hydrocarbon radical, either saturated or unsaturated, linear or branched, in particular comprising from 1 to 20, preferably from 1 to 14 carbon atoms, and more particularly chosen from alkyl radicals, preferably from C₁ to C₁₀, aryl radicals, preferably from C₆ to C₁₂, and aralkyl radicals, preferably from C₇ to C₁₄.

Thus, when the catalyst (C2), preferably selected from metal hydrides, organometallic compounds and organometallic hydrides, comprises at least one of the above-mentioned ligands, the metal of the catalyst is not only bonded to at least one hydrogen atom and/or to at least one hydrocarbon radical (R), but also to at least one of these ligands, e.g. to the O atom of the oxo radical by a double bond, or to the O atom of the alkoxo, aryloxo or aralkyloxo radical (OR') by a single bond, or to the N atom of the nitrido radical by a triple bond, or to the N atom of the imido radical (NR") by a double bond, or to the N atom of the amido radical (NR¹R²) by a single bond. Generally, the presence of the oxo, alkoxo, aryloxo, aralkyloxo, nitrido, imido and/or amido ligands in the catalyst (C2) can favourably influence the behaviour of the catalyst in the ethane metathesis of stage (3).

In addition, the catalyst (C2) chosen from metal hydrides, organometallic compounds and organometallic hydrides is preferably supported on, more particularly grafted onto, a solid support that can be chosen from metal or refractory oxides, sulfides, carbides, nitrides and salts, and from carbon, mesoporous materials, organic/inorganic hybrid materials, metals and molecular sieves. The solid support can be chosen from silicas, aluminas, silica-aluminas and aluminium silicates, either simple or modified by other metals, and from zeolites, clays, titanium oxide, cerium oxide, magnesium oxide, niobium oxide, tantalum oxide and zirconium oxide. It can be preferably chosen from silicas, aluminas, silica-aluminas and aluminium silicates, either simple or modified by other metals. The catalyst (C2) is preferably a metal hydride or an organometallic hydride grafted onto a solid support, more especially chosen from niobium hydrides or organometallic niobium hydrides grafted onto silica, tantalum hydrides or organometallic tantalum hydrides grafted onto silica, molybdenum hydrides or organometallic molybdenum hydrides grafted onto silica, tungsten hydrides or organometallic tungsten hydrides grafted onto alumina, and rhenium hydrides or organometallic rhenium hydrides grafted onto silica.

The catalyst (C2) selected from metal hydrides, organometallic compounds and organometallic hydrides can be in particular chosen from the metal catalysts described in International Patent Applications WO 98/02244, WO 03/061823 and WO 2004/089541, and prepared according to any of the methods described in said Applications.

It is observed that stage (3) leads to the formation of a mixture (M2) comprising the higher alkanes (C₃₊), preferably (C₄₊), methane and optionally unreacted ethane and where applicable propane (in particular when the higher alkanes (C₄₊) are preferably prepared, instead of the higher alkanes (C₃₊)).

Without wishing to be bound by any theory relating to reaction mechanisms, it may be thought that stage (3) involves a plurality of simultaneous reactions leading finally to the mixture (M2). In stage (3), it is observed that ethane can react with itself in contact with the catalyst (C2), and produce in particular methane and propane by an ethane metathesis reaction, such as a self-metathesis reaction, in particular according to the following equation (8):

2C₂H₆ → CH₄ + C₃H₈ (8)

It is furthermore observed that stage (3) can involve another ethane metathesis reaction, such as a cross-metathesis reaction, in particular between ethane and the propane obtained previously and optionally resulting from the unreacted C₃₊ alkanes of the natural gas such as the (C₃₋₆) alkanes. This reaction can produce in particular methane and butane(s), according to the following equation (9):

C₂H₆ + C₃H₈ → CH₄ + C₄H₁₀ (9)

It is furthermore observed that stage (3) can also involve other similar ethane metathesis reactions, in particular other similar cross-metathesis reactions between ethane and successively higher alkanes having at least 4 carbon atoms, previously obtained by other preceding cross-metathesis reactions and/or optionally resulting from the unreacted C₃₊ alkanes of the natural gas such as the (C₃₋₆) alkanes. These reactions can produce in particular methane and higher alkanes having one additional carbon atom compared with the higher alkanes involved in the reactions, for example according to the following equations (10) to (13):

C₂H₆ + C₄H₁₀ → CH₄ + C₅H₁₂ (10)

C₂H₆ + C₅H₁₂ → CH₄ + C₆H₁₄ (11)

C₂H₆ + C₆H₁₄ → CH₄ + C₇H₁₆ (12)

C₂H₆ + C₇H₁₆ → CH₄ + C₈H₁₈ (13)

It is thus found that in the process of the invention, ethane appears to be an essential intermediate alkane which allows ultimately for natural gas to be converted easily into the higher alkanes (C₃₊), preferably (C₄₊). It is remarkable to note that the present process can be carried out with a high overall yield, particularly when methane produced in stage (3) is separated and isolated from the mixture (M2) and is then preferably recycled into stage (1). Metathesis reactions between (C₃₊) or (C₄₊) alkanes and other (C₃₊) or (C₄₊) alkanes are also possible in stage (3) leading to formation of higher alkanes.

Stage (3) thus leads to the formation of a mixture (M2) comprising the higher alkanes (C₃₊), preferably (C₄₊), methane and optionally unreacted ethane and where applicable propane. Under the operating conditions of stage (3), the mixture (M2) is generally obtained in gaseous form or in mixed gaseous/liquid form.

Stage (3) can be advantageously performed in conditions suited to promoting the development of the ethane metathesis reactions, such as those described above, in particular towards an optimum production of the higher alkanes (C₃₊), preferably (C₄₊). Thus, stage (3) can be performed at a temperature chosen in a range of from 20 to 400°C, preferably from 50 to 350 °C, particularly from 70 to 300 °C, more especially from 100 to 250 °C. It can also be performed under an absolute total pressure chosen in a range of from 0.1 to 50 MPa, preferably from 0.1 to 30 MPa, particularly from 0.1 to 20 MPa. It can also be performed in the presence of at least one inert agent, either liquid or gaseous, in particular of at least one inert gas preferably chosen from nitrogen, helium and argon. It can also be performed in the presence of hydrogen, in particular of the hydrogen separated and isolated from the mixture (M1) in stage (2), more particularly under a partial pressure of hydrogen chosen in a range of from 0.0001 to 10 MPa, preferably from 0.001 to 1 MPa.

Stage (3) can be performed in various ways, either intermittently, or discontinuously, or preferably continuously, in a reaction zone (Z2) which can be either distinct and separate from fractionation zones (F1) and (F2) wherein stages (2) and (4) are respectively performed, e.g. in at least one reactor, or arranged in a part of said fractionation zone (F1) or preferably of said fractionation zone (F2), e.g. in at least one distillation column reactor, such as described in American Patent US 4,242,530.

In stage (3), it is possible, for example, to add ethane (or the mixture (M1)) and optionally one or more unreacted C₃₊ alkanes of the natural gas such as the (C₃₋₆) alkanes to the catalyst (C2). It is also possible to add the catalyst (C2) to ethane (or to the mixture (M1)) and optionally to said C₃₊ alkane(s). It is also possible to add ethane (or the mixture (M1)), optionally said C₃₊ alkane(s) and the catalyst (C2) simultaneously into the reaction zone (Z2) wherein stage (3) is performed. It is also possible to add ethane (or the mixture (M1)) and optionally said C₃₊ alkane(s) separately to the catalyst (C2), in a given chronological order or in a reverse order.

The reaction zone (Z2) can be of a type identical to or different from that of the reaction zone (Z1) of stage (1). Thus, for example, the reaction zone (Z2) can comprise at least one reactor chosen from static reactors, recycling reactors and dynamic reactors. More particularly, stage (3) can be performed in a static reactor containing fixed quantities of catalyst (C2), of ethane (or of the mixture (M1)) and optionally of unreacted C₃₊ alkane(s) of the natural gas. Stage (3) can also be performed in a recycling reactor in which ethane (or the mixture (M1)), optionally one or more unreacted C₃₊ alkanes of the natural gas such as the (C₃₋₆) alkanes, and/or at least one component of the mixture (M2) can be recycled. Stage (3) can also be performed in a dynamic reactor containing the catalyst (C2), in particular in the form of solid particles or of a bed, through which ethane (or the mixture (M1)) and optionally one or more unreacted C₃₊ alkanes of the natural gas passes or circulates.

In practice, stage (3) can be performed in a reaction zone (Z2) comprising at least one reactor chosen from tubular (or multi-tubular) reactors, distillation column reactors, slurry reactors, fluidised bed reactors, mechanically agitated bed reactors, fluidised and mechanically agitated bed reactors, fixed bed reactors, moving bed reactors and circulating bed reactors. The catalyst (C2) is generally solid, and can be optionally mixed with an inert solid agent chosen in particular from silicas, aluminas, silica-aluminas and aluminium silicates, either simple or modified by other metals. It can be arranged inside the tube(s) of a tubular (or multi-tubular) reactor. It can also be arranged inside a distillation column reactor, wherein the catalyst is preferably a component of a distillation system functioning as both a catalyst and a distillation packing, i.e. a packing for a distillation column having both a distillation function (as in stage (2) or preferably (4)) and a catalytic function (as in stage (3)): for example, rings, saddles, balls, irregular shapes, granulates, sheets, tubes, spirals, packed in bags, as described in American Patent US 4,242,530. The catalyst (C2) can also form the bed of a fluidised and/or mechanically agitated bed, a fixed bed, a moving bed or a circulating bed of said reactors. Ethane (or the mixture (M 1)), preferably in admixture with one or more unreacted C₃₊ alkanes of the natural gas, can be introduced into one of said reactors, preferably continuously, and generally can pass or circulate into the tube(s), or through the bed or the distillation packing of said reactors. In order to promote the development of the reactions towards an optimum production of the higher alkanes (C₃₊), preferably (C₄₊), stage (3) can advantageously be performed by withdrawing the higher alkanes (C₊₃), preferably (C₄₊), and/or the methane out of the reaction zone (Z2), preferably continuously.

Thus, for example, stage (3) can be performed:
(i) by continuously introducing ethane (or the mixture (M1)) and optionally one or more unreacted C₃+ alkanes of the natural gas into the reaction zone (Z2) containing the catalyst (C2), so as to form the mixture (M2) comprising methane, the higher alkanes (C₃₊), preferably (C₄₊), and optionally unreacted ethane and where applicable propane, and
(ii) by continuously withdrawing at least a part of the mixture (M2) out of the reaction zone (Z2), so as to subject said part of the mixture (M2) to stage (4) for separating the higher alkanes (C₃₊), preferably (C₄₊), from the mixture (M2) and isolating said higher alkanes from the rest of the mixture, and preferably to return said rest of the mixture, particularly unreacted ethane and where applicable propane into the reaction zone (Z2), in particular so as to pursue the ethane metathesis.

The process then comprises a stage (4) comprising separating the higher alkanes (C₃₊), preferably (C₄₊), from the mixture (M2), so as to isolate said higher alkanes. Stage (4) can be performed in various ways, either intermittently, or discontinuously, or preferably continuously, in a fractionation zone (F2). It can comprise one or more methods of fractionation of the mixture (M2), in particular chosen from any known methods or combinations of method for fractionation of such a mixture, preferably chosen from the following six types of fractionation:
- fractionation by change of physical state, preferably of gaseous/liquid phase of the mixture, in particular by distillation or by condensation (or liquefaction), more particularly by partial condensation (or partial liquefaction), e.g. by means of distillation/condensation column or tower,
- fractionation by molecular filtration, preferably by means of semi-permeable and selective membrane,
- fractionation by adsorption, preferably by means of molecular sieve or any other adsorbent,
- fractionation by absorption, preferably by means of absorbing oil,
- fractionation by cryogenic expansion, preferably by means of expansion turbine, and
- fractionation by compression, preferably by means of gas compressor, so as in particular to separate and to isolate the higher alkanes (C₃₊), preferably (C₄₊), and optionally methane, unreacted ethane and where applicable propane, from the mixture (M2).

Any method that is useful in general for separating the higher alkanes (C₃₊), preferably (C₄₊) from methane, optionally ethane and where applicable propane, can be utilized in stage (4).

Stage (4) thus makes it possible to separate and to isolate, from the mixture (M2), the higher alkanes (C₃₊), preferably (C₄₊). It can also permit to separate and to isolate methane, which can be preferably recycled into stage (1). Furthermore, it can also permit that unreacted ethane and where applicable propane is/are separated and isolated, and then preferably is/are recycled into stage (3) for ethane metathesis, in particular together or separately

Stage (4) can be performed in a fractionation zone (F2) which is either distinct and separate from the reaction zone (Z2) where stage (3) is performed, e.g. in one or more distillation/condensation column or tower, or arranged in a part of said reaction zone (Z2), e.g. in at least one distillation column reactor, such as described in American Patent US 4,242,530.

Thus, a distillation column reactor can be advantageously used both for stage (3) and stage (4), and contain the catalyst (C2). More particularly, the catalyst (C2) can be arranged inside the distillation column reactor, as a component of a distillation system functioning as both a catalyst and a distillation packing. Thus, stages (3) and (4) can be simultaneously performed in at least one distillation column reactor comprising both a reaction zone and a fractionation zone. In the reaction zone, the contacting of ethane and preferably of one or more unreacted C₃₊ alkanes of the natural gas, or optionally of the mixture (M1) with the catalyst (C2) can be performed as in stage (3), so as to form the mixture (M2) preferably in a gaseous/liquid form. Simultaneously in the fractionation zone, the separation of the higher alkanes (C₃₊), preferably (C₄₊), from the mixture (M2) can be performed as in stage (4), in particular by distillation or by condensation, more particularly by partial condensation, so as to separate and to isolate said higher alkanes.

More generally, a fractionation of the mixture (M2) can be performed by change of the physical state, preferably of the gaseous/liquid phase of the mixture, e.g. in at least one distillation/condensation column or tower, or in a distillation column reactor, in particular by refrigeration or cryogenic processes. Preferably it can be performed by at least one cooling of the gaseous mixture (M2) to at least one temperature at which at least one component of the mixture condenses and the rest of the mixture remains in gaseous form, so as to condense, to separate and to isolate said the component(s) from said rest of the mixture.

Generally, a fractionation of the mixture (M2) can also be performed by molecular filtration of the mixture, preferably by passing the gaseous mixture (M2) through at least one semi-permeable and selective membrane, so as to arrest and to isolate at least one component of the mixture and to allow the rest of the mixture to pass through.

Generally, a fractionation of the mixture (M2) can also be performed by adsorption, preferably by passing the gaseous mixture (M2) onto at least one molecular sieve or any other adsorbent, so as to retain at least one component of the mixture and to allow the rest of the mixture to pass through, and then by submitting the component(s) thus retained to at least one desorption step, preferably by the TSA or the PSA method, so as to isolate said component(s).

Generally, a fractionation of the mixture (M2) can also be performed by absorption, preferably by contacting the gaseous mixture (M2) with an absorbing oil, so as to soak up at least one component of the mixture, to form an oil/component mixture and to allow the rest of the mixture to be free and not absorbed, and then by subjecting said oil/component mixture to a temperature above the boiling point of the component, but below that of the oil, so as to separate and to isolate the component from the oil.

Generally, a fractionation of the mixture (M2) can also be performed by cryogenic expansion, preferably by dropping the temperature of the gaseous mixture (M2) to a temperature so as to condense at least one component of the mixture while maintaining the rest of the mixture in gaseous form, with the help of an expansion turbine which rapidly expands the gaseous mixture and causes a significant drop of the temperature.

Generally, a fractionation of the mixture (M2) can also be performed by compression, preferably by compressing the gaseous mixture (M2), e.g. by means of a gas compressor, so as to condense at least one component of the mixture, to separate and to isolate it from the rest of the gaseous mixture.

For example, a fractionation of the gaseous mixture (M2) can be performed by change of the physical state, preferably of the gaseous/liquid phase of the mixture, e.g. in at least one distillation/condensation column or tower, or in a distillation column reactor, in particular by cooling of the gaseous mixture (M2) to a temperature at which the higher alkanes (C₃₊) preferably (C₄₊) condense, so as to condense said higher alkanes, to separate and to isolate them in liquid form from the rest of the gaseous mixture comprising methane and optionally unreacted ethane and where applicable propane.

For example, a fractionation of the gaseous mixture (M2) can also be performed by molecular filtration, preferably by passing the gaseous mixture (M2) through at least one semi-permeable and selective membrane, so as to arrest and to isolate the higher alkanes (C₃₊) preferably (C₄₊), and to allow the rest of the gaseous mixture comprising methane and optionally unreacted ethane and where applicable propane to pass through.

The semi-permeable and selective membranes can be chosen from any membrane suitable for arresting and separating the higher alkanes (C₃₊), preferably (C₄₊) from methane, optionally ethane and where applicable propane. They can be preferably chosen from zeolite membranes, porous alumina membranes, ceramic membranes, flowing liquid membranes and supported liquid membranes.

For example, a fractionation of the gaseous mixture (M2) can also be performed by adsorption, preferably by passing the gaseous mixture onto at least one molecular sieve or any other adsorbent, so as to retain the higher alkanes (C₃₊), preferably (C₄₊), and to allow the rest of the gaseous mixture comprising methane and optionally unreacted ethane and where applicable propane to pass through, and by submitting said higher alkanes thus retained to at least one desorption step, preferably by the TSA or the PSA method, so as to isolate them.

The adsorbents can be chosen from any adsorbent suitable for retaining and separating the higher alkanes (C₃₊), preferably (C₄₊) from methane, optionally ethane and where applicable propane. They can be preferably chosen from molecular sieves, silica gels, activated charcoals and activated carbons.

For example, a fractionation of the gaseous mixture (M2) can also be performed by absorption, preferably by contacting the gaseous mixture (M2) with an absorbing oil, e.g. in an absorption tower, so as to soak up the higher alkanes (C₃₊), preferably (C₄₊), and to form an oil/higher alkane (C₃₊) or (C₄₊) mixture, and then by subjecting said mixture, e.g. in a recovery tower, to a temperature above the boiling point of the higher alkanes (C₃₊) or (C₄₊), but below that of the oil, so as separate and to isolate said higher alkanes from the oil.

For example, a fractionation of the gaseous mixture (M2) can also be performed by cryogenic expansion, preferably by dropping the temperature of the gaseous mixture (M2) in particular by use of external refrigerants to cool the gaseous mixture and then by means of an expansion turbine which rapidly expands the chilled gaseous mixture and causes the temperature to drop significantly, so as to condense the higher alkanes (C₃₊), preferably (C₄₊), to separate and to isolate them in liquid form from the rest of the gaseous mixture comprising methane, optionally unreacted ethane and where applicable propane.

For example, a fractionation of the gaseous mixture (M2) can also be performed by compression, preferably by compressing the gaseous mixture (M2) e.g. by means of a gas compressor, so as to condense the higher alkanes (C₃₊), preferably (C₄₊), to separate and to isolate them in liquid form from the rest of the gaseous mixture comprising methane, optionally unreacted ethane and where applicable propane.

Stage (4) can also be performed by a double (or multiple) fractionation of the mixture (M2), comprising a combination of two (or more) successive fractionations of an identical or different type, preferably chosen from the six above-mentioned types of fractionation, so as in particular to separate and to isolate (a) the higher alkanes (C₃₊), preferably (C₄₊), (b) methane and (c) unreacted ethane and where applicable propane. Methane can be advantageously separated and isolated in stage (4), and then preferably recycled into stage (1). In addition, it may also be particularly advantageous that in stage (4), ethane and where applicable propane is/are separated and isolated, and then preferably is/are recycled into stage (3) for ethane metathesis, in particular together or separately. The separation, isolation and recycling of the methane are particularly advantageous in the process of the invention, since methane is produced in large quantities in stage (3), and its recycling into stage (1) improves enormously the overall output of the process. The same applies to the separation, isolation and recycling of the unreacted ethane and where applicable propane, since their recycling into stage (3) for ethane metathesis improves substantially the output of said stage.

For example, it is possible to perform a double fractionation by subjecting the gaseous mixture (M2) comprising the higher alkanes (C₃₊), preferably (C₄₊), methane, unreacted ethane and where applicable propane, to a combination of two successive fractionations of one and the same type, namely by change of the physical state, preferably of the gaseous/liquid phase of the mixture, e.g. in one or more distillation/condensation columns or towers, or in a distillation column reactor, as follows:
(i) by a first cooling of the gaseous mixture (M2) to a temperature at which the higher alkanes (C₃₊) preferably (C₄₊) condense, so as to condense said higher alkanes, to separate and to isolate them in liquid form from the rest of the gaseous mixture comprising in methane, unreacted ethane and where applicable propane, and
(ii) by a second cooling of said rest of the gaseous mixture to a temperature at which ethane and where applicable propane condense(s), so as to condense the unreacted ethane and where applicable the propane, and to separate and to isolate it (or them) in liquid form from methane which is preferably isolated in its turn in gaseous form.

For example, it is also possible to perform a double fractionation by subjecting the above-mentioned gaseous mixture (M2) to a combination of two successive fractionations of one and the same type, for example by molecular filtration:
(i) by passing the gaseous mixture (M2) through a first semi-permeable and selective membrane, so as to arrest and to isolate the higher alkanes (C₃₊), preferably(C₄₊), and to allow the rest of the gaseous mixture comprising methane, unreacted ethane and where applicable propane to pass through, and
(ii) by passing said rest of the gaseous mixture through a second semi-permeable and selective membrane, so as to arrest and to isolate the unreacted ethane and where applicable the propane, and to allow methane to pass through and preferably to be isolated in its turn in gaseous form.

For example, it is also possible to perform a double fractionation by subjecting the above-mentioned gaseous mixture (M2) to a combination of two successive fractionations of one and the same type, namely by adsorption:
(i) by passing the gaseous mixture (M2) onto a first molecular sieve or any other adsorbent, so as to retain the higher alkanes (C₃₊), preferably (C₄₊), and to allow the rest of the gaseous mixture comprising methane, unreacted ethane and where applicable propane to pass through, then
(ii) by passing said rest of the gaseous mixture onto a second molecular sieve or any other adsorbent, so as to retain the unreacted ethane and where applicable the propane, and to allow methane to pass through and preferably to be isolated in gaseous form, and
(iii) by respectively submitting the higher alkanes (C₃₊), preferably (C₄₊) and the unreacted ethane and where applicable the propane thus retained, to desorption steps, in particular by the TSA or the PSA method, so as to isolate said higher alkanes and the unreacted ethane and where applicable the propane.

For example, it is also possible to perform a double fractionation by subjecting the above-mentioned gaseous mixture (M2) to a combination of two successive fractionations of one and the same type, namely by adsorption, e.g. in one or more absorption towers:
(i) by contacting the gaseous mixture (M2) with a first absorbing oil, so as to soak up the higher alkanes (C₃₊), preferably (C₄₊), to form an oil/higher alkanes (C₃₊) or (C₄₊) mixture, and to allow the rest of the gaseous mixture comprising methane, unreacted ethane and where applicable propane to be free in gaseous form,
(ii) by contacting said rest of the gaseous mixture with a second absorbing oil, so as to soak up unreacted ethane and where applicable propane, to form an oiUethane/(propane) mixture, and to allow methane to be free in gaseous form and preferably to be separated and isolated, and
(iii) by subjecting said oil/higher alkanes (C₃₊) or (C₄₊) mixture, e.g. in a recovery tower, to a temperature above the boiling point of said higher alkanes, but below that of the oil, so as to separate and to isolate said higher alkanes from the oil, and preferably by subjecting said oil/ethane/(propane) mixture, e.g. in a recovery tower, to a temperature above the boiling point of ethane and where applicable of propane, but below that of the oil, so as to separate and to isolate the unreacted ethane and where applicable the propane from the oil.

For example, it is also possible to perform a double fractionation by subjecting the above-mentioned gaseous mixture (M2) to a combination of two successive fractionations of one and the same type, namely by cryogenic expansion:
(i) by dropping the temperature of the gaseous mixture (M2) by means of an expansion turbine, so as to condense the higher alkanes (C₃₊), preferably (C₄₊), to separate and to isolate them in liquid form from the rest of the gaseous mixture comprising methane, unreacted ethane and where applicable propane, and
(ii) by dropping the temperature of said rest of the gaseous mixture by means of an expansion turbine, so as to condense the unreacted ethane and where applicable the propane, to separate and to isolate it (or them) in liquid form from methane which is preferably isolated in its turn in gaseous form.

For example, it is also possible to perform a double fractionation by subjecting the above-mentioned gaseous mixture (M2) to a combination of two successive fractionations of one and the same type, namely by compression:
(i) by compressing the gaseous mixture (M2), e.g. by means of a gas compressor, so as to condense the higher alkanes (C₃₊), preferably (C₄₊), to separate and to isolate them in liquid form from the rest of the gaseous mixture comprising methane, unreacted ethane and where applicable propane, and
(ii) by compressing said rest of the gaseous mixture, e.g. by means of a gas compressor, so as to condense the unreacted ethane and where applicable the propane, to separate and to isolate it (or them) in liquid form from methane which is preferably isolated in its turn in gaseous form.

It is also possible to perform a double (or multiple) fractionation by subjecting the above-mentioned gaseous mixture (M2) to a combination of two successive fractionations of a different type, preferably chosen from the six above-mentioned types of fractionation.

More particularly, a double fractionation of the above-mentioned mixture (M2) can be performed by combining two successive fractionations of a different type, namely:
- the one by change of the physical state, preferably of the gaseous/liquid phase of the mixture, in particular by distillation or by condensation, and the other by molecular filtration, preferably by means of a semi-permeable and selective membrane, or
- the one by change of the physical state, preferably of the gaseous/liquid phase of the mixture, in particular by distillation or by condensation, and other by adsorption, preferably by means of a molecular sieve or any other adsorbent, or
- the one by change of the physical state, preferably of the gaseous/liquid phase of the mixture, in particular by distillation or by condensation, and the other by absorption, preferably by means of an absorbing oil, or
- the one by change of the physical state, preferably of the gaseous/liquid phase of the mixture, in particular by distillation or by condensation, and the other by cryogenic expansion, preferably by means of an expansion turbine, or
- the one by change of the physical state, preferably of the gaseous/liquid phase of the mixture, in particular by distillation or by condensation, and the other by compression, preferably by means of a gas compressor, or
- the one by molecular filtration, preferably by means of a semi-permeable and selective membrane, and the other by adsorption, preferably by means of a molecular sieve or any other adsorbent, or
- the one by molecular filtration, preferably by means of a semi-permeable and selective membrane, and the other by absorption, preferably by means of an absorbing oil, or
- the one by molecular filtration, preferably by means of a semi-permeable and selective membrane, and the other by cryogenic expansion, preferably by means of an expansion turbine, or
- the one by molecular filtration, preferably by means of a semi-permeable and selective membrane, and the other by compression, preferably by means of a gas compressor, or
- the one by adsorption, preferably by means of a molecular sieve or any other adsorbent, and the other by absorption, preferably by means of an absorbing oil, or
- the one by adsorption, preferably by means of a molecular sieve or any other adsorbent, and the other by cryogenic expansion, preferably by means of an expansion turbine, or
- the one by adsorption, preferably by means of a molecular sieve or any other adsorbent, and the other by compression, preferably by means of a gas compressor, or
- the one by absorption, preferably by means of an absorbing oil, and the other by cryogenic expansion, preferably by means of an expansion turbine, or
- the one by absorption, preferably by means of an absorbing oil, and the other by compression, preferably by means of a gas compressor, or
- the one by cryogenic expansion, preferably by means of an expansion turbine, and the other by compression, preferably by means of a gas compressor.

Each of said combinations can be performed in a given chronological order or in a reverse order.

Thus, for example, two successive fractionations of a different type can be performed advantageously:
(i) by cooling of the gaseous mixture (M2) to a temperature at which the higher alkanes (C₃₊) preferably (C₄₊) condense, so as to condense said higher alkanes, to separate and to isolate them in liquid form from the rest of the gaseous mixture comprising methane, unreacted ethane and where applicable propane, and
(ii) by passing said rest of the gaseous mixture through a semi-permeable and selective membrane, so as to arrest the unreacted ethane and where applicable the propane, and to allow methane to pass through and preferably to be isolated in its turn in gaseous form.

For example, it is also possible to perform the two above-mentioned successive fractionations, but in a reverse order, that is to say:
(i) by passing the gaseous mixture (M2) through a semi-permeable and selective membrane, so as to arrest and to isolate the higher alkanes (C₃₊) preferably (C₄₊), and to allow the rest of the gaseous mixture comprising methane, unreacted ethane and where applicable propane to pass through, and
(ii) by cooling said rest of the gaseous mixture to a temperature at which ethane and where applicable propane condense(s), so as to condense the unreacted ethane and where applicable the propane, to separate and to isolate it (or them) in liquid form from methane which is preferably isolated in its turn in gaseous form.

Thus, e.g. by means of a double (or multiple) fractionation such as one of those described above, methane can be separated and isolated in stage (4), and then preferably recycled into stage (1). In addition, unreacted ethane and where applicable propane is/are preferably separated and isolated in stage (4), and then advantageously is/are recycled into stage (3), in particular together or separately.

The process of the invention has the advantage of converting the natural gas easily and with a high yield into the higher alkanes (C₃₊), preferably (C₄₊), which are capable of being easily transportable in liquid form, in conditions relatively close to standard temperature and pressure conditions. It also exhibits a very high "carbon atom efficiency" which can be equal to or higher than 95%. Furthermore, it can advantageously produce great amounts of hydrogen which can be isolated and then supply sufficient thermal and/or electrical energies for running the present process.

The following example illustrates the present invention.

### Example

### (1) Preparation of a metal catalyst (C1): an organometallic tantalum hydride grafted onto silica.

In a first stage, 50 mg of a silica sold under the trade name "Aerosil 200"® by Degussa (Germany) were subjected to a dehydroxylation treatment at 700°C, under an absolute pressure of 10⁻² Pa, for 15 hours. At the end of this time, the silica was cooled to 25°C.

In a second stage, 50 mg of the silica prepared beforehand were isolated and introduced into a reactor at 25°C under an argon atmosphere. A precursor (Pr) was then introduced into the reactor, namely tantalum tris(neopentyl)neopentylidene, of general formula (14):

Ta [- CH₂ - C(CH₃)₃]₃ [ = CH - C(CH₃)₃] (14)

The reactor was then heated to 70°C and maintained at this temperature for 2 hours, so as to sublime the precursor (Pr) onto the silica and to form an organometallic tantalum compound grafted onto the silica. At the end of this time, the surplus of unreacted precursor (Pr) was removed by inverse sublimation at 70°C. The reactor was then cooled to 25°C and the organometallic tantalum compound grafted onto the silica was isolated under an argon atmosphere. It contained 5.5 wt % of tantalum.

In a third stage, the organometallic tantalum compound grafted onto the silica thus prepared beforehand was subjected to a hydrogenolysis treatment by contacting at 150°C said compound with hydrogen, under an absolute hydrogen pressure of 73 kPa, for 15 hours. At the end of this time, a metal catalyst (C1) based on an organometallic tantalum hydride grafted onto silica and containing 5.5 wt % of tantalum was obtained and isolated under an argon atmosphere.

### (2) Preparation of a metal catalyst (C2): an organometallic tungsten hydride grafted onto alumina.

In a first stage, 2 g of an α-alumina (alpha-alumina) containing 90 wt % of alumina and 9 wt % of water, and sold by Johnson Matthey (Great Britain), were subjected to a calcination treatment at 500°C, under a dry air current, for 15 hours, then to a dehydroxylation treatment at 500°C, under an absolute pressure of 10⁻² Pa, for 15 hours. At the end of this time, the alumina was cooled under an argon atmosphere to 25°C.

In a second stage, 2 g of the alumina thus prepared beforehand were isolated and introduced at 25°C under an argon atmosphere into a reactor fitted with a means of agitation. After this, 305 mg of a precursor (Pr'), namely tungsten tris(neopentyl)neopentylidyne of general formula (15):

W [- CH₂ - C(CH₃)₃]₃[=C- C(CH₃)₃] (15)

were introduced into the reactor under an argon atmosphere.

The reactor was heated to 66°C and maintained at this temperature and under agitation for 4 hours. At the end of this time, the reactor was cooled to 25°C, and the solid mixture was then washed with n-pentane at 25°C. The solid compound thus washed was vacuum dried, then isolated under an argon atmosphere, so as to obtain an organometallic tungsten compound grafted onto alumina and containing 3.9 wt % of tungsten.

In a third stage, 40 mg of the organometallic tungsten compound grafted onto alumina thus prepared beforehand were isolated and subjected to a hydrogenolysis treatment by contacting at 150°C said compound with hydrogen under an absolute hydrogen pressure of 73 kPa, for 15 hours. At the end of this time, the reactor was cooled to 25°C, and there was obtained and isolated under an argon atmosphere a metal catalyst (C2) based on an organometallic tungsten hydride grafted onto alumina and containing 3.9 wt % of tungsten.

### (3) Conversion of natural gas into higher alkanes (C₄₊).

In a preliminary stage, a natural gas was subjected to a purification treatment in order to eliminate essentially carbon dioxide and hydrogen sulfide.

According to stage (1), the natural gas thus treated was introduced continuously into a reactor (R1) heated to 400°C, under an absolute pressure of 5 MPa and containing the catalyst (C1) prepared beforehand. In the reactor (R1) there formed a gaseous mixture (M1) comprising ethane, hydrogen and unreacted methane and unreacted C₃₊ alkanes of the natural gas namely propane, butanes, pentanes and hexanes. The mixture (M1) was withdrawn continuously out of the reactor (R1).

According to stage (2), the gaseous mixture (M1) obtained in stage (1) was introduced continuously into a fractionation zone (F1) wherein ethane in an admixture with the unreacted C₃₊ alkanes of the natural gas, namely propane, butanes, pentanes and hexanes, was separated and isolated, while methane and hydrogen were also separated and isolated. One part of the hydrogen was used as an agent of activation or regeneration of the catalysts (C1) and (C2), and the other as a fuel for producing thermal and electrical energy directly employed in the present process. The methane thus separated was recycled continuously into the reactor (R1).

According to stage (3), ethane in admixture with the unreacted C₃₊ alkanes was introduced continuously into a reactor (R2) heated to 150°C, under an absolute pressure of 2 MPa and containing the catalyst (C2) prepared beforehand. In the reactor (R2), there formed a mixture (M2) comprising the higher alkanes (C₄₊), namely butanes, pentanes, hexanes, heptanes and octanes, methane and unreacted ethane and propane. The gaseous, mixture (M2) was withdrawn continuously out of the reactor (R2).

According to stage (4), the mixture (M2) thus obtained in stage (3) was introduced continuously into a fractionation zone (F2) wherein the higher alkanes (C₄₊) were separated and isolated, while methane and unreacted ethane in admixture with propane were also separated and isolated. Unreacted ethane in admixture with propane was recycled continuously into the reactor (R2), while methane was continuously recycled into the reactor (R1).

## Claims

1. Process for converting natural gas into higher alkanes having at least 3 carbon atoms (C₃₊), preferably at least 4 carbon atoms (C₄₊), **characterised in that** it comprises the following stages:
- a stage (1) comprising contacting natural gas with a metal catalyst (C1) capable of producing, in contact with alkane, reactions involving the splitting and recombining of carbon-carbon and/or carbon/hydrogen and/or carbon metal bonds, so as to form a mixture (M1) comprising ethane and hydrogen,
- optionally a stage (2) comprising separating ethane from the mixture (M1), so as to isolate the ethane,
- a stage (3) for ethane metathesis, comprising contacting the ethane isolated in stage (2) or the mixture (M1) obtained in stage (1) with a catalyst (C2) capable of producing, in contact with alkane, reactions involving the splitting and recombining of carbon-carbon and/or carbon-hydrogen and/or carbon-metal bonds, said catalyst being identical to or different from the catalyst (C1), so as to form a mixture (M2) comprising higher alkanes (C₃₊), preferably (C₄₊), and methane, and
- a stage (4) comprising separating the higher alkanes (C₃₊), preferably (C₄₊), from the mixture (M2), so as to isolate said higher alkanes.

2. Process according to claim 1, **characterised in that** prior to stage (1), natural gas is subjected to a purification stage for removing poison(s) capable of deactivating the metal catalyst(s) (C1) and/or (C2) and optionally nitrogen (N₂), preferably by removing one or more poisons chosen from water, carbon dioxide and sulfur compounds especially selected from hydrogen sulfide, carbonyl sulfide and mercaptans, and optionally nitrogen (N₂).

3. Process according to claim 1 or 2, **characterised in that** the catalysts (C1) and (C2) are chosen from catalysts capable of alkane metathesis, preferably chosen from supported metal clusters and more particularly chosen from metal hydrides, organometallic compounds and organometallic hydrides, preferably supported on, in particular grafted onto, a solid support.

4. Process according to claim 3, **characterized in that** the supported metal clusters comprise one or more metals of Groups 4, 5 and/or 6 of the Periodic Table of the Elements, preferably tantalum.

5. Process according to claim 3 or 4, **characterized in that** the supported metal clusters comprise a solid support and a metal cluster, preferably bonding between the metals of the metal cluster, and bonding between the metal cluster and the solid support.

6. Process according to claim 5, **characterized in that** the metal atom of the metal cluster is bonded to at least one other metal of the metal cluster, preferably to six or fewer metal atoms of the metal cluster.

7. Process according to any one of claims 3 to 6, **characterized in that** the supported metal clusters comprise a solid support chosen from a metal oxide, a refractory oxide, a molecular sieve, a zeolite, a metal phosphate and a material comprising a metal and oxygen, preferably silica.

8. Process according to claim 3, **characterised in that** the catalysts chosen from metal hydrides, organometallic compounds and organometallic hydrides comprise at least one metal chosen from lanthanides, actinides and metals of Groups 2 to 12, preferably transition metals of Groups 3 to 12 of the Periodic Table of the Elements.

9. Process according to claim 8, **characterized in that** the catalysts comprise at least one metal chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium, preferably from yttrium, titanium, zirconium, hafnium, vanadium, niobium, tantalum, chromium, molybdenum, tungsten, rhenium, ruthenium and platinum, more particularly from niobium, tantalum, molybdenum, tungsten and rhenium.

10. Process according to claim 8 or 9, **characterised in that** the catalysts comprise a solid support chosen from metal or refractory oxides, sulfides, carbides, nitrides and salts, and from carbon, mesoporous materials, organic/inorganic hybrid materials, metals and molecular sieves.

11. Process according to any one of claims 1 to 10, **characterised in that** stage (1) is performed at a temperature chosen in a range of from 50 to 700 °C, preferably from 70 to 650 °C, in particular from 100 to 600 °C.

12. Process according to any one of claims 1 to 11, **characterised in that** stage (1) is performed under an absolute total pressure chosen in a range of from 0.1 to 100 MPa, preferably from 0.1 to 50 MPa, more particularly from 0.1 to 30 MPa.

13. Process according to any one of claims 1 to 12, **characterised in that** stage (1) is performed under a partial pressure of methane chosen in a range of from 0.1 to 50 MPa, preferably from 0.1 to 30 MPa, more particularly from 0.2 to 20 MPa, in particular from 0.5 to 10 MPa.

14. Process according to any one of claims 1 to 13, **characterised in that** the mixture (M1) comprises ethane and hydrogen, and optionally one or more unreacted natural gas components, particularly unreacted methane and one or more unreacted C₃₊ alkanes of the natural gas, and **in that** optional stage (2) comprises separating from the mixture (M1) ethane preferably together with one or more said unreacted C₃₊ alkanes, so as to isolate the ethane in admixture with said unreacted C₃₊ alkanes, and preferably to use the ethane in such an admixture subsequently in stage (3).

15. Process according to any one of claims 1 to 14, **characterised in that** stage (2) comprises one or more fractionations of the mixture (M1), preferably chosen from: fractionation by change of physical state, preferably of gaseous/liquid phase of the mixture, in particular by distillation or by condensation, more particularly by partial condensation, especially by means of distillation/condensation column or tower, fractionation by molecular filtration, preferably by means of semi-permeable and selective membrane,
fractionation by adsorption, preferably by means of molecular sieve or any other adsorbent, fractionation by absorption, preferably by means of absorbing oil,
fractionation by cryogenic expansion, preferably by means of expansion turbine, and fractionation by compression, preferably by means of gas compressor.

16. Process according to any one of claims 1 to 15, **characterised in that** the mixture (M1) comprises in addition unreacted methane which is preferably separated and isolated from the mixture (M1) in stage (2), and then is particularly recycled into stage (1).

17. Process according to any one of claims 1 to 16, **characterised in that** in stage (2), hydrogen is separated and isolated from the mixture (M1), and is then preferably used in one or more applications or stages, distinct or not from the process, in particular used as an agent of activation or regeneration of the catalyst(s) (C1) and/or (C2), or as a fuel for producing thermal or electrical energy, in particular as a fuel in hydrogen fuel cells for producing electrical energy, such energies being preferably used in the process, or as a fuel for automobile, or as a reagent in a chemical, petrochemical or refinery plant.

18. Process according to any one of claims 1 to 17, **characterised in that** stage (3) comprises contacting ethane and one or more unreacted C₃₊ alkanes of the natural gas isolated from the mixture (M1) in stage (2) with the catalyst (C2).

19. Process according to any one of claim 1 to 18, **characterised in that** stage (3) is performed at a temperature chosen in a range of from 20 to 400 °C, preferably from 50 to 350°C, more particularly from 70 to 300 °C, in particular from 100 to 250 °C.

20. Process according to any one of claims 1 to 19, **characterised in that** stage (3) is performed under a total absolute pressure chosen in a range of from 0.1 to 50 MPa, preferably from 0.1 to 30 MPa, more particularly from 0.1 to 20 MPa.

21. Process according to any one of claims 1 to 20, **characterised in that** stage (3) is performed in the presence of hydrogen, preferably under a partial pressure of hydrogen chosen in a range of from 0.0001 to 10 MPa, preferably from 0.001 to 1 MPa, more particularly with hydrogen separated and isolated from the mixture (M1) in stage (2).

22. Process according to any one of claims 1 to 21, **characterised in that** stage (4) comprises one or more fractionations of the mixture (M2), preferably chosen from:
- fractionation by change of physical state, preferably of gaseous/liquid phase of the mixture, in particular by distillation or by condensation, more particularly by partial condensation, especially by means of distillation/condensation column or tower,
- fractionation by molecular filtration, preferably by means of semi-permeable and selective membrane,
- fractionation by adsorption, preferably by means of molecular sieve or any other adsorbent,
- fractionation by absorption, preferably by means of absorbing oil,
- fractionation by cryogenic expansion, preferably by means of expansion turbine, and
- fractionation by compression, preferably by means of gas compressor.

23. Process according to any one of claims 1 to 22, **characterised in that** methane separated and isolated from the mixture (M2) in stage (4) is recycled into stage (1).

24. Process according to any one of claims 1 to 23, **characterised in that** the mixture (M2) in addition comprises unreacted ethane and where applicable propane which is/are separated and isolated from the mixture (M2) in stage (4), and then preferably is/are recycled into stage (3), in particular together or separately
